# EUROPEAN PATENT SPECIFICATION

(11) **EP 0 828 473 B1**
(45) Date of publication and mention of the grant of the patent: **09.07.2003**
(21) Application number: 96908068.8
(22) Date of filing: 15.03.1996
(51) Int. Cl.: A61K 7/00, A61K 7/48

(54) **A COSMETIC PRODUCT TO PREVENT AND CORRECT SKIN DAMAGE**
KOSMETISCHE ZUBEREITUNG ZUR PRÄVENTION UND BESEITIGUNG VON HAUTSCHÄDEN
PRODUITS COSMETIQUE CON U POUR PREVENIR OU CORRIGER UNE DETERIORATION DE LA PEAU

(30) Priority: 26.05.1995 US 451940
(43) Date of publication of application: 18.03.1998
(73) Proprietor: UNILEVER N.V., 3013 AL Rotterdam (NL); UNILEVER PLC, London EC4P 4BQ (GB)
(72) Inventor: SUARES, Alan, Joseph, Cheshire, CT 06410 (US); NETTESHEIM, Susan, New York, NY 10023 (US); INDURSKY, Michael, Fairfield, CT 06430 (US); BERTOLINI, Peter, Shelton, CT 06484 (US)
(74) Representative: Rots, Maria Johanna Francisca
(86) International application number: EP9601168
(87) International publication number: WO96037179

(56) References cited:
- EP-A- 0 345 082
- EP-A- 0 501 714
- WO-A-94/06405
- CH-A- 585 647
- DE-A- 3 911 089
- GB-A- 2 231 782
- NL-A- 9 301 506
- US-A- 5 306 486

## Description

The invention concerns a cosmetic product formed with separate containers storing skin damage prevention and correction compositions.

### The Related Art

Dual purpose single formulation cosmetic products are quite handy and popular. Examples of 2-in-1 commercial formulations are cleanser & moisturizer and shampoo & conditioner products. Unfortunately, single formulations often compromise the performance of the severally combined actives.

A response to this problem has been the development of skin treatment regimes. Two or more different cosmetic compositions are employed in these regimes and applied to the skin in sequential order. Illustrative commercial treatment regime compositions are cleansers, moisturizers, toners and facial foundations.

The present invention has sought a system to deliver actives for prevention of skin damage and also to correct damage. Herein it is suggested that separate compositions in a treatment regime be formulated for skin damage prevention and for correction. Each of these compositions would then require respective timing for application to skin. Prevention compositions are suggested for daytime use utilizing sunscreens as the active. Correction compositions would be for nighttime use. They should incorporate an α-hydroxycarboxylic acid as the active. Relatively high levels of the α-hydroxycarboxylic acids must be present in the correction composition. By contrast, little or none of this substance is necessary for the prevention composition during daytime use in the sun.

When a skin treatment regime requires multi-composition use, there have been problems with consumer education and discipline. Education is particularly a problem in mass market outlets where a cosmetic knowledgeable sales staff is generally absent. A customer may purchase one product in a treatment regime but may be uninformed with respect to a necessary complementary regime product. Even a somewhat educated customer may select the wrong complementary product. Most frustrating is when a totally educated customer discovers that the store either does not carry the complementary product or is temporarily out of stock.

Even when a customer has been educated and supplied with proper products, the products may become separated at home. Moreover, the necessary reinforcement of use according to a regime may no longer be present.

Accordingly, it is an object of the present invention to provide a cosmetic product for a skin treatment regime that assists the consumer in maintaining the regime.

It is another object of the present invention to provide a cosmetic product with multi-compositions for a skin treatment regime that ensures the recommended compositions are all provided to the consumer in a single sale.

Still another object of the present invention is to provide a cosmetic product for a skin treatment regime that daily serves as a reminder to the consumer as to the proper utilization of component compositions.

Yet another object of the present invention is to provide a cosmetic product for a skin treatment regime that maintains each of the compositions together in a unit to avoid separation and misplacement within a consumer's home.

### SUMMARY OF THE INVENTION

A cosmetic product to prevent and correct skin damage is provided including: a first composition having a sunscreen present in an effective amount to prevent UV radiation from penetrating the composition to reach a user's skin; a second composition having a C₂-C₃₀ α-hydroxycarboxylic acid or salt thereof present in an effective amount to correct UV radiation induced skin damage;a first container for storing the first composition; and a second container for storing the second composition, the first and second containers being releasably joined together.

Preferably the first and second containers are each fitted with a mechanism for coupling the two together, with a preferable coupling being a screw thread arrangement. Advantageously the first and second containers are jars stacked one above the other and releasably locked together through the coupling mechanism.

Also provided is a method to prevent and correct skin damage, the method including the steps of:
providing a first composition having a sunscreen present in an effect amount to prevent UV radiation from penetrating the composition to reach a user's skin;
providing a second composition having a C₂-C₃₀ α-hydroxycarboxylic acid or salt thereof present in an effective amount to correct UV radiation induced skin damage; storing the first composition in a first container; storing the second composition in a second container, the first and second containers being releasably joined together; applying the first composition to the skin so as to remain in contact during daytime and to prevent UV radiation induced skin damage; and applying the second composition after application of the first composition so as to remain in contact over night on the skin to correct for any UV radiation damage.

### BRIEF DESCRIPTION OF THE DRAWING

The above features, advantages and objects of the present invention will more fully be appreciated through the following detailed discussion, reference being made to the sole Figure showing an exploded view of a stacked first and second container screwably and releasably joinable together.

### DETAILED DESCRIPTION

Now it has been found that a day and night skin treatment regime with respective damage prevention and correction compositions can be sold to a consumer as a unit, serve as a reminder for joint usage and also educate a customer in proper application thereof. These advantages are achieved through disposition and sale of the respective compositions in respective containers wherein the two containers are joined together. Most preferably, joinder is achieved through a stacking arrangement of the containers as respective jars releasably lockable one on top another.

The Figure illustrates the dispensing package as including a first container or jar **2**, a second container or jar **4** and a domed cap **6**. First container **2** is fitted at an open end thereof with a threaded male screw **8** that can lockingly engage cap **6** for closure of that jar. A first composition incorporating a sunscreen for use as a damage prevention formulation during daytime wear is stored within container **2**.

Second container **4** at an open end thereof is also fitted with a threaded male screw **10**. A second composition incorporating an α-hydroxycarboxylic acid or salt thereof is stored-within this second container. Threaded male screw **10** is lockingly joinable with a threaded female screw **12** arranged below a closed bottom end **14** of the first container **2.** In a preferred embodiment, the first container **2** storing the prevention composition has an outer color different from that of the outer color of the second container **4.** Most preferred is that the first container **2** be colored predominantly white (day formula) and the second container **4** be colored predominantly black (night formula). The term "predominantly" means greater than 50% of the surface area of outer walls of the container. Color coding provides the consumer with an educational signal for proper use of the cosmetic product.

Beyond the package, a first essential element of cosmetic products according to the present invention is that of a first composition incorporating a sunscreen. The term "sunscreen" is used to denote ultraviolet ray-blocking compounds inhibiting absorption within the wavelength region between 290 and 420 nm. These compounds may either be organic or inorganic. The organic compounds are preferred. When the sunscreen is inorganic and serves as the sole sun protective substance, it should be present at levels ranging from 5 to 30%, preferably from 8 to 15% by weight.

Typical inorganic sunscreens include titanium dioxide, zinc oxide, iron oxide and combinations thereof. Most preferred is titanium dioxide, especially having an average particle size no higher than 700 nm, preferably no higher than 200 nm, optimally less than 35 nm.

Organic sunscreens suitable for the present invention may be classified into five groups based upon their chemical structures: para-amino benzoates; salicylates; cinnamates; benzophenones; coumarins; azoles and miscellaneous chemicals including menthyl anthralinate. Also polymeric particles may be useful such as polyethylene and polyamides. Among FDA-approved sunscreens are those listed in the Table below.

| UV-A Absorbers | Approved % |
|---|---|
| **Oxybenzone,** also known as 2-hydroxy-4-methoxy benzophenone, and benzophenone-3, available as Uvinul M-40 and Gafsorb 2H4M | 2-6 |
| **Butyl Methoxydibenzoylmethane** N/A available as Parsol 1789 | |
| **Dioxybenzone,** also known as 2,2 dihydroxy-4-methoxy benzophenone, and benzophenone-8 | 3 |
| **Sulibenzone,** also known as 2-hydroxy-4-methoxy benzophenone-5-sulphonic acid, and benzophenone-4, available as Uvinul MS-40 and Gafsorb 2H4MS | 5-10 |
| **Menthyl anthralinate,** also known as menthyl-o-aminobenzoate | 3.5-5 |

| UV-B Absorbers | Approved % |
|---|---|
| **p-Amino benzoic acid,** also known as PABA | 5-15 |
| **Amyl dimethyl PABA** (NA), also known as amyl-p-dimethyl ammonium benzoate, available as Padimate A | 1-5 |
| **2-Ethoxy ethyl p-methoxy cinnamate** (NA), available as Cinoxate and Givtan-F | 1-3 |
| Diethanolamine p-ethoxy cinnamate, also known as DEA methoxy cinnamate, available as Parsol-Hydro | 8-10 |
| **Digalloyl trioleate** (NA), a component of Solprotex I | 2-5 |
| **Ethyl-4-bis (hydroxypropyl) aminobenzoate,** also known as ethyl dihydroxy propyl PABA, available as Amerscreen P | 1-5 |
| **2-Ethyl hexyl-2-cyano-3,3 diphenyl acrylate,** also known as octocrylene and available as Uvinul N-539 | 7-10 |
| **Ethyl hexyl p-methoxy cinnamate,** also known as octyl methoxycinnamate available as Parsol MCX | 2-7.5 |
| **2-Ethyl hexyl salicylate,** also known as octyl salicylate | 3-5 |
| Glyceryl aminobenzoate, also known as glyceryl p-aminobenzoate and glyceryl PABA, available as Escalol 106 | 2-3 |
| Homomenthyl salicylate, also known as 3,3,5-trimethylcyclohexyl salicylate | 4-15 |
| **Lawsone with dihydroxyacetone** (NA) | 0.25 with 33 |
| **Octyl dimethyl PABA,** also known as 2-ethyl hexyl p-dimethyl p-aminobenzoate, and 2-ethyl hexyl dimethyl PABA, available as Padimate O and Escalol 507 | 1.4-8 |
| **2-Phenyl benzimidazole 5-sulphoic acid** | 1.4 |
| **Triethanolamine salicylate** | 5-12 |

Organic sunscreen compound will range in amount anywhere from 0.1 to 25%, optimally from 1 to 15%, most preferably from 5 to 10% by weight.

Another essential element of cosmetic products according to the present invention is that of a second composition which incorporates a C₂-C₃₀ α-hydroxycarboxylic acid or salt thereof. A wide variety of α-hydroxycarboxylic acids may be employed, suitable examples of which include:
α-hydroxyethanoic acid
α-hydroxypropanoic acid
α-hydroxyhexanoic acid
α-hydroxyoctanoic acid
α-hydroxydecanoic acid
α-hydroxydodecanoic acid
α-hydroxytetradecanoic acid
α-hydroxyhexadecanoic acid
α-hydroxyoctadecanoic acid
α-hydroxyeicosanoic acid
α-hydroxydocosanoic acid
α-hydroxyhexacosanoic acid, and
α-hydroxyoctacosanoic acid

Particularly preferred from the above list are α-hydroxyethanoic acid (commonly known as glycolic acid), α-hydroxypropanoic acid (commonly known as lactic acid) and α-hydroxyoctanoic acid (commonly known as α-hydroxycaprylic acid or HCA).

For purposes of this invention, the term α-hydroxycarboxylic acids are intended to include not only the acid form but also salts thereof. Typical salts are the alkalimetal, ammonium and C₂-C₆₀ ammonium salts thereof. Particularly preferred are the sodium, potassium, triethanolammonium, polyethyleneimine and ammonium salts. Combinations of all the foregoing may be present in the compositions. Amounts of the α-hydroxycarboxylic acid or salt thereof may range from about 0.01 to about 15%, preferably from about 0.1 to about 12%, optimally from about 0.5 to about 9% by weight.

Either or both of the first and second compositions of the present invention may be solid or liquid, aqueous or anhydrous and opaque or transparent.

A pharmaceutically acceptable carrier is ordinarily utilized to deliver the sunscreen and the α-hydroxycarboxylic acid active components of the first and second compositions.
Most preferably, cosmetic compositions of this invention will be in emulsion form. By definition, an emulsion is a dispersed system containing at least two immiscible liquid phases, one of which is dispersed in the form of small droplets throughout the other. Water and oil are the most common immiscible phases. An emulsion in which oil is dispersed as droplets throughout the aqueous phase is termed an oil-in-water emulsion. When water is the dispersed phase and an oil is the dispersion medium, a water-in-oil emulsion exists. Contemplated within the scope of this invention are emulsions in the form of lotions and creams of both types of emulsions, those where the water phase is continuous and those where the oil phase is continuous. The amount of these phases may range from 99:1 to 1:99 by weight.

The term "pharmaceutically acceptable carrier" according to this invention includes emollients, surfactants, humectants and water. Total amount of the carrier may range from 30 to 99.9%, preferably from 50 to 90%, optimally from 70 to 85% by weight.

A variety of oily emollients may be employed in the compositions of this invention. These emollients may be selected from one or more of the following classes:
1. Hydrocarbon oils and waxes. Examples thereof are mineral oil, petrolatum, paraffin, ceresin, ozokerite, microcrystalline wax, polyethylene, and perhydrosqualene.
2. Triglyceride esters such as vegetable and animal fats and oils. Examples include castor oil, cocoa butter, safflower oil, cottonseed oil, corn oil, olive oil, cod liver oil, almond oil, avocado oil, palm oil, sesame oil, squalene, and soybean oil.
3. Acetoglyceride esters, such as acetylated monoglycerides.
4. Ethoxylated glycerides, such as ethoxylated glyceryl monostearate.
5. Alkyl esters of fatty acids having 10 to 22 carbon atoms. Methyl, isopropyl, and butyl esters of fatty acids are useful herein. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
6. Alkenyl esters of fatty acids having 10 to 22 carbon atoms. Examples thereof include oleyl myristate, oleyl stearate, and oleyl oleate.
7. Fatty acids having 10 to 22 carbon atoms. Suitable examples include pelargonic, lauric, myristic, palmitic, stearic, isostearic, hydroxystearic, oleic, linoleic, ricinoleic, arachidic, behenic, and erucic acids.
8. Fatty alcohols having 10 to 22 carbon atoms. Lauryl, myristyl, cetyl, hexadecyl, stearyl, isostearyl, hydroxystearyl, oleyl, ricinoleyl, behenyl, erucyl, and 2-octyl dodecanyl alcohols are examples of satisfactory fatty alcohols.
9. Fatty alcohol ethers. Ethoxylated fatty alcohols of 10 to 22 carbon atoms including the lauryl, cetyl, stearyl, isostearyl, oleyl, and cholesterol alcohols, having attached thereto from 1 to 50 ethylene oxide groups or 1 to 50 propylene oxide groups,
10. Ether-esters such as fatty acid esters of ethoxylated fatty alcohols.
11. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases are illustrative of emollients derived from lanolin.
12. Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 mono- oleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid esters, sorbitan fatty acid esters, and polyoxyethylene sorbitan fatty acid esters are satisfactory polyhydric alcohol esters.
13. Wax esters such as beeswax, spermaceti, myristyl myristate, stearyl stearate.
14. Beeswax derivatives, e.g. polyoxyethylene sorbitol beeswax. These are reaction products of beeswax with ethoxylated sorbitol of varying ethylene oxide content, forming a mixture of ether esters.
15. Vegetable waxes including carnauba and candelilla waxes.
16. Phospholipids such as lecithin and derivatives.
17. Sterols. Cholesterol, cholesterol fatty acid esters are examples thereof.
18. Amides such as fatty acid amides, ethoxylated fatty acid amides, solid fatty acid alkanolamides.

Amounts of the above listed emollients may range anywhere from about 0.5 to about 80% by weight of the total composition. Preferably the amounts of these emollients will range from about 1 to about 25%, optimally between about 5 and 15% by weight.

Humectants of the polyhydric alcohol-type may also be included in the compositions of this invention. The humectant aids in increasing the effectiveness of the emollient, reduces scaling, stimulates removal of built-up scale and improves skin feel. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably glycerol. The amount of humectant may range anywhere from 0.1 to 40%, preferably between 1 and 15% by weight of the composition.

For improved lubricity, there may also be included one or more silicone oils or fluids which may be selected from a dimethyl polysiloxane, a methylphenyl polysiloxane and an alcohol-soluble silicone glycol copolymer. Preferred siloxanes include dimethyl polysiloxane (CTFA name: dimethicone), a polysiloxane end-blocked with trimethyl units and polydimethylcyclosiloxane, (CTFA name: cyclomethicone). The preferred siloxanes exhibit a viscosity from about 2 to 50 centistokes at 25°C. Amounts of the silicones can conveniently range from 0.1 to 80% by weight of the compositions, preferably from 1 to 20% by weight.

Surfactants can also be included in the compositions of this invention. These may be selected from nonionic, anionic, cationic or amphoteric emulsifying agents. They may suitably range in amount anywhere from 0.1 to 30% by weight. Illustrative of the nonionic surfactants are alkoxylated compounds based upon C₈-C₂₂ fatty alcohols, C₈-C₂₂ fatty acids and sorbitan. Particularly preferred is Tween 80®, an ethoxylated sorbitan (CTFA name: POE (2) sorbitan monooleate), and Myrj 59® which is a polyethoxylated C₁₈ fatty acid (CTFA name: POE (100) stearate). A preferred non-ethoxylated nonionic surfactant is Arlacel 60®, (CTFA name: sorbitan monostearate).

Both first and second compositions of the invention can also include thickeners/viscosifiers in amounts from 0.01 to 10% by weight of the composition. As known to those skilled in the art, the precise amount of thickeners can vary depending upon the consistency and thickness of the composition which is desired. Exemplary thickeners are magnesium aluminum silicate (Veegum®), guar gums (such as Jaguar HP-120®), xanthan gum, sodium carboxymethyl cellulose, hydroxyalkyl and alkyl celluloses, and cross-linked acrylic acid polymers such as those sold by B.F. Goodrich under the Carbopol® trademark.

Waterproofing agents may be included along with the sunscreen containing compositions of this invention. These agents may range in amount anywhere from about 0.5 to 10% by weight. Common waterproofing agents are polymers and copolymers based on PVP and acrylic or methacrylic esters. Specific examples are PVP/Hexadecene Copolymer (Ganex V-216®), PVP/Eicosene Copolymer (Ganex V-220®), PVP/Ethyl Methacrylate/Methacrylic Acid Copolymer, Ammonium Acrylates Copolymer, and Polyolprepolymer-2 (ex Penederm/Barnet).

Preservatives can desirably be incorporated into both the first and second compositions of this invention to protect against the growth of potentially harmful microorganisms. While it is in the aqueous phase that microorganisms tend to grow, microorganisms can also reside in the oil phase. As such, preservatives which have solubility in both water and oil are preferably employed in the present compositions. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are methyl paraben, imidazolidinyl urea, sodium dehydroxyacetate, propyl paraben and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

When present, the amount of water in a composition may range anywhere from 1 to 99%, preferably from 20 to 90%, optimally between 40 and 70% by weight.

Minor adjunct ingredients may also include fragrances, antifoam agents, herbal extracts, opacifiers and colorants, each in their effective amounts to accomplish their respective functions.

Vitamins may also be included in the compositions of the present invention. Especially preferred is Vitamin A palmitate (retinyl palmitate) and Vitamin E linoleate (tocopheryl linoleate). Other esters of Vitamins A and E may also be utilized.

In a preferred aspect of the present invention, the first and second compositions will each have at least ten components in common. Moreover, the ten components will be present at essentially identical concentrations in both the first and second compositions.

The following examples will more fully illustrate the composition embodiments of this invention. All parts, percentages and proportions referred to herein and in the appended claims are by weight unless otherwise indicated.

### EXAMPLE 1

A pair of prevent and correct compositions were prepared according to the following formulations.

**TABLE I**

| **PREVENT COMPOSITION** | |
|---|---|
| **COMPONENT** | **WEIGHT %** |
| Parsol MCX® | 4.00 |
| Stearic Acid | 3.00 |
| Finsolv TN® | 3.00 |
| Butylene Glycol | 2.00 |
| Glycerin | 2.00 |
| MYRJ 59® | 2.00 |
| Uvinul M-40® | 2.00 |
| Crodamol ISNP® | 2.00 |
| Glycerol Monostearate | 1.50 |
| Stearyl Alcohol | 1.50 |
| Glycolic Acid (70%) | 1.40 |
| Triethanolamine (99%) | 1.20 |
| Lactic Acid | 1.15 |
| Magnesium Aluminum Silicate | 1.00 |
| Arlacel 60® | 1.00 |
| Silicone 1401® | 0.80 |
| Disodium EDTA | 0.50 |
| Jaguar HP-120® | 0.50 |
| Cholesterol | 0.30 |
| Ammonia (Aqueous 26BE) | 0.30 |
| Fragrance | 0.30 |
| Tween 80® | 0.30 |
| Polyethyleneimine | 0.25 |
| Methylparaben | 0.15 |
| Vitamin E Acetate | 0.10 |
| Propylparaben | 0.10 |
| Hydroxycaprylic Acid | 0.10 |
| Vitamin A Palmitate | 0.10 |
| Water | qs |

**TABLE II**

| **CORRECT COMPOSITION** | |
|---|---|
| **COMPONENT** | **WEIGHT %** |
| Glycolic Acid (70%) | 5.74 |
| Butylene Glycol | 3.00 |
| Hetester® FAO/Fine | 3.00 |
| Stearic Acid | 3.00 |
| Finsolv TN® | 2.50 |
| Ceraphyl 230® | 2.50 |
| Ammonia (Aqueous 26BE) | 2.20 |
| Glycerin | 2.00 |
| Myrj 59® | 2.00 |
| Polyethylene Imine | 2.00 |
| Stearyl Alcohol | 1.50 |
| Glycerl Monostearate | 1.50 |
| Triethanolamine (99%) | 1.20 |
| Magnesium Aluminum Silicate | 1.00 |
| SE 700 | 1.00 |
| Silicone Fluid 10 | 1.00 |
| Arlacel 60® | 1.00 |
| Dow Corning 1401® | 0.80 |
| Jaguar HP-120® | 0.50 |
| Disodium EDTA | 0.50 |
| Sodium Stearoyl Lactylate | 0.50 |
| Cholesterol | 0.30 |
| Fragrance | 0.30 |
| Tween 80® | 0.30 |
| Methylparaben | 0.15 |
| Antifoam Emulsion | 0.10 |
| Vitamin E Acetate | 0.10 |
| Propylparaben | 0.10 |
| Hydroxycaprylic Acid | 0.10 |
| Vitamin A Palmitate | 0.10 |
| Bisabolol | 0.10 |
| Water | qs |

### EXAMPLE 2

The Prevent and Correct dual composition product was tested for consumer acceptability in a shaved leg sting evaluation with a consumer panel. Formulations tested were identical to that described in Example 1, except for the changes in α-hydroxycarboxylic acid concentrations as noted below.

**TABLE III**

| **COMPOSITION** | **WEIGHT %** |
|---|---|
| Prevent | 1% glycolic, 1% lactic, sunscreen (SPF 8) |
| Correct | 4% glycolic (no sunscreen) |
| Prevent/Correct (All-in-One Composition) | 5% glycolic, 1% lactic, sunscreen (SPF 8) |

An expert panel of subjects were selected for the test, each being qualified and proven to show sensitivity to product differences. Subjects were provided with two charged syringes, each filled with 2 cc of sample composition and clearly labelled with left/right designations. Subjects shaved their legs in their shower using a wet razor and applied sample composition immediately upon drying off. They took note of sting sensations and filled out a standard questionnaire.
Results of the test are recorded below.

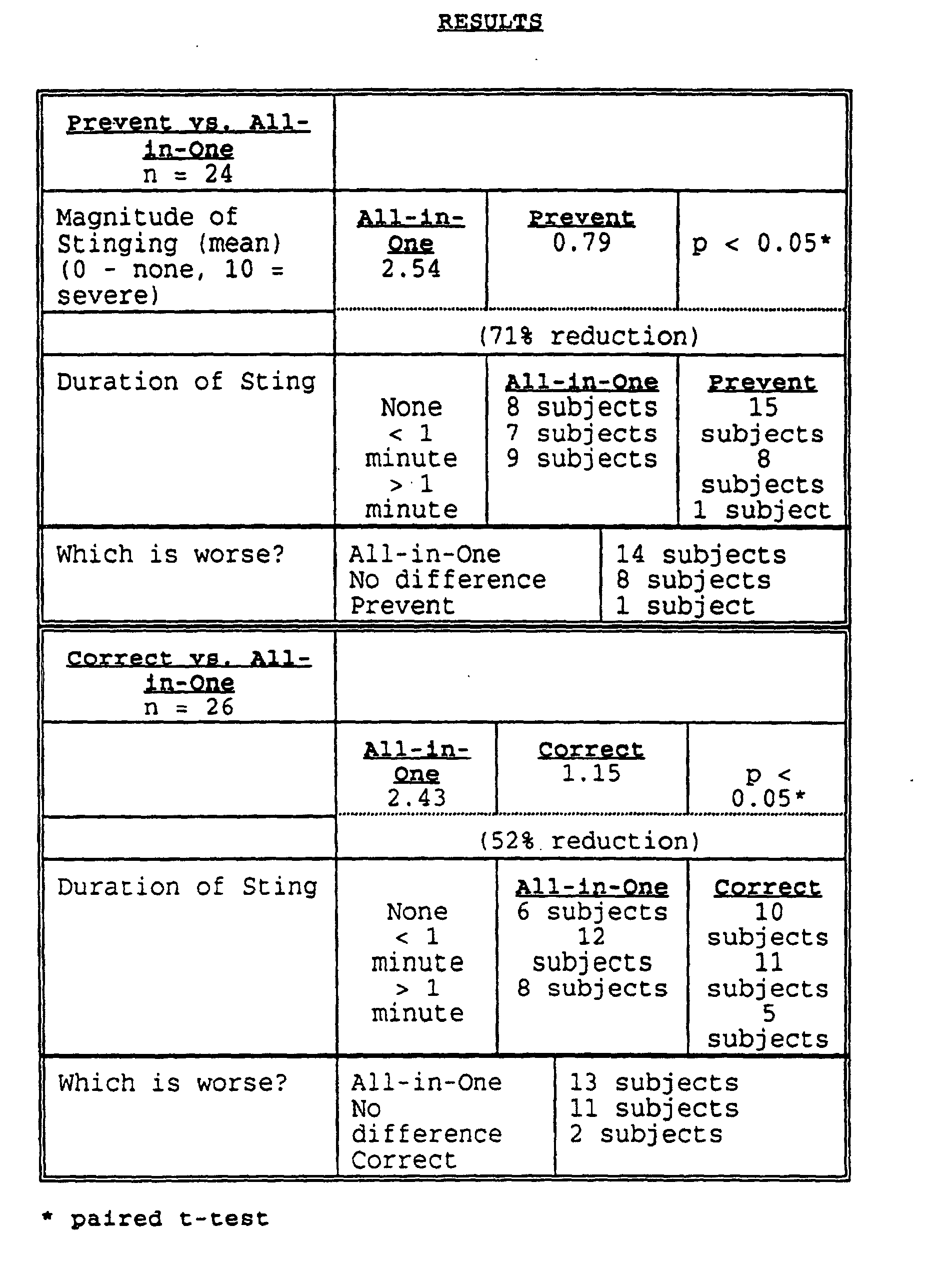

Sequential usage of the Prevent and Correct compositions delivered a daily dose of 6% α-hydroxycarboxylic acid with sunscreen level to provide SPF 8. The comparison product where the Prevent and Correct formulations were combined into a single "all-in-one" composition also contained 6% α-hydroxycarboxylic acid with sunscreen level to provide SPF 8.

The clinical study demonstrated that use of the separate Prevent and Correct compositions phased-in the delivery of α-hydroxycarboxylic acid thereby reducing the sting/consumer discomfort that arises when both formulas are blended together as a combined single composition. Sting reduction of 71% and 52% were obtained, respectively in comparison of the Prevent and Correct compositions against the combination all-in-one product.

### EXAMPLE 3

Another pair of prevent and correct compositions illustrative of the present invention are outlined below. The prevent composition will have an SPF of at least 21.

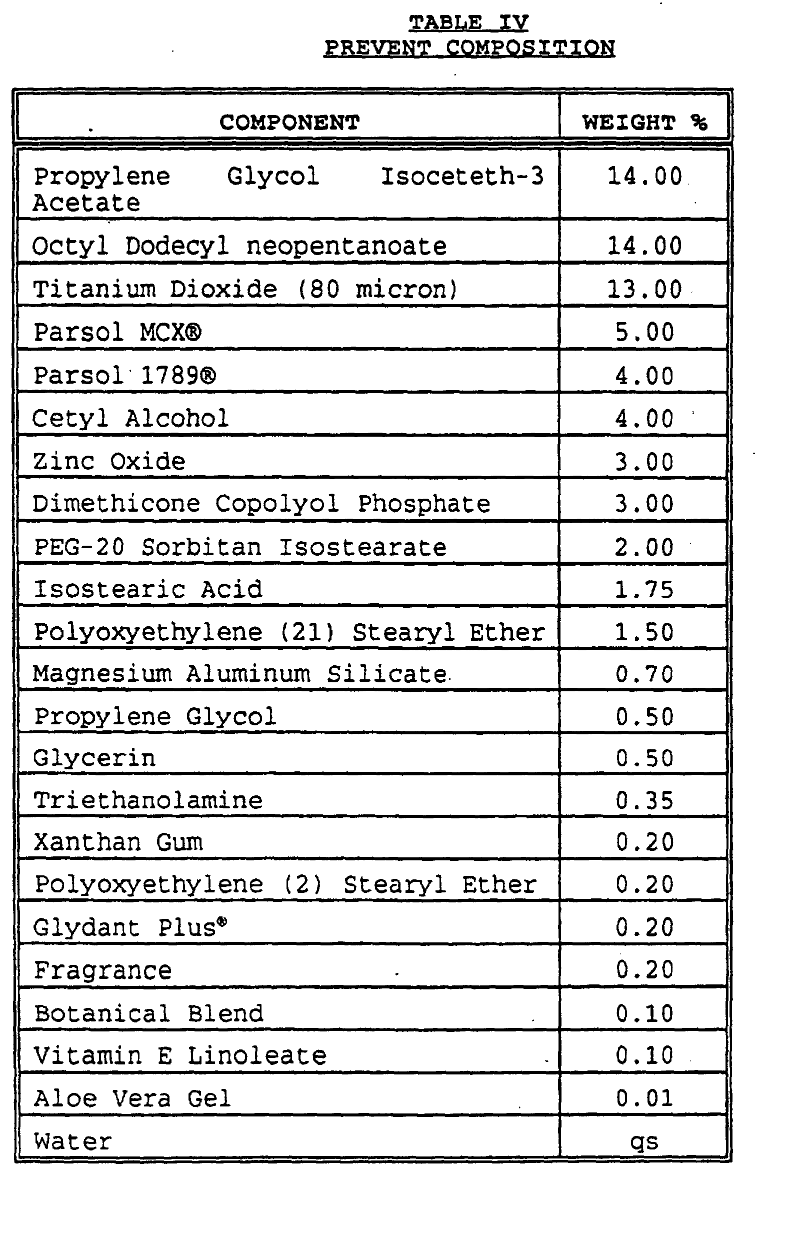

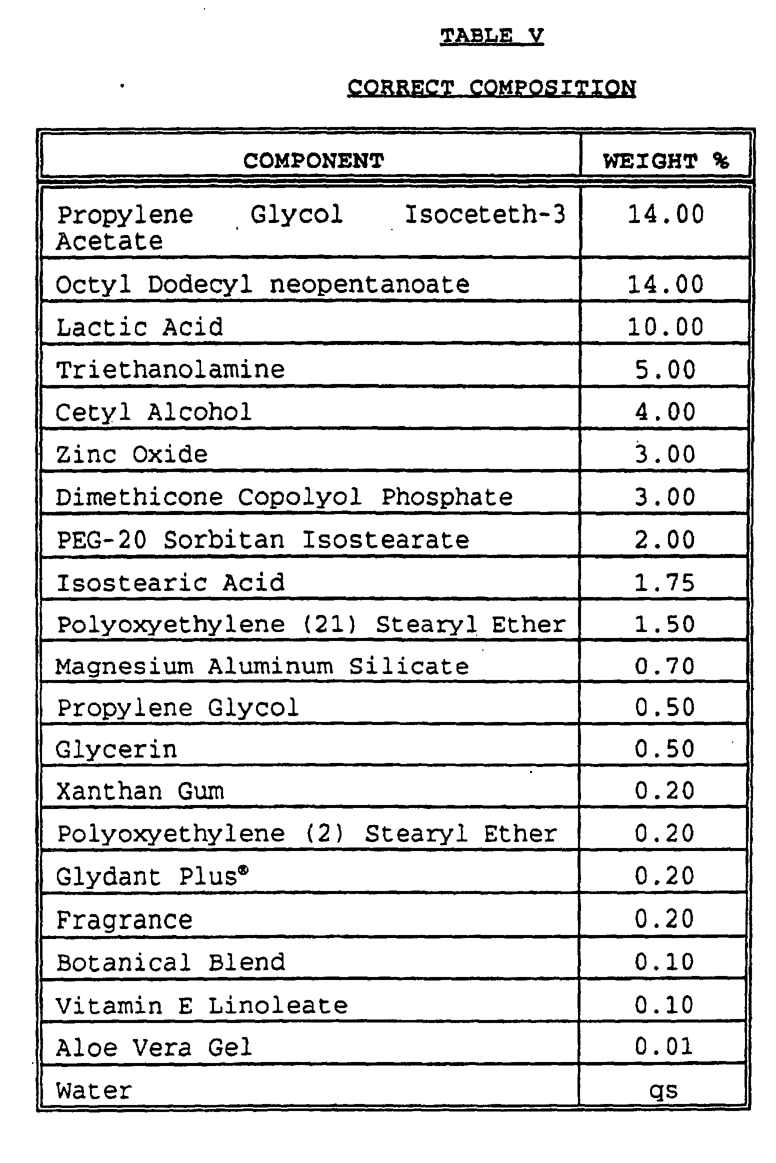

### EXAMPLE 4

A further pair of prevent and correct compositions according to the present invention are prepared from the following components.

**TABLE VI**

| **PREVENT COMPOSITION** | |
|---|---|
| **COMPONENT** | **WEIGHT %** |
| Ethylhexyl p-Methoxycinnamate | 7.00 |
| Glycerin | 4.00 |
| Oxybenzone | 3.00 |
| Alkyl Polyglycoside | 3.00 |
| Cetyl Alcohol | 2.50 |
| Glycerl Monostearate | 2.00 |
| Octyl Palmitate | 2.00 |
| Silicone Fluid | 1.50 |
| Petroleum Jelly | 1.00 |
| Methyl Paraben | 0.15 |
| Propyl Paraben | 0.10 |
| Fragrance | 0.10 |
| Antifoam | 0.01 |
| Water | qs |

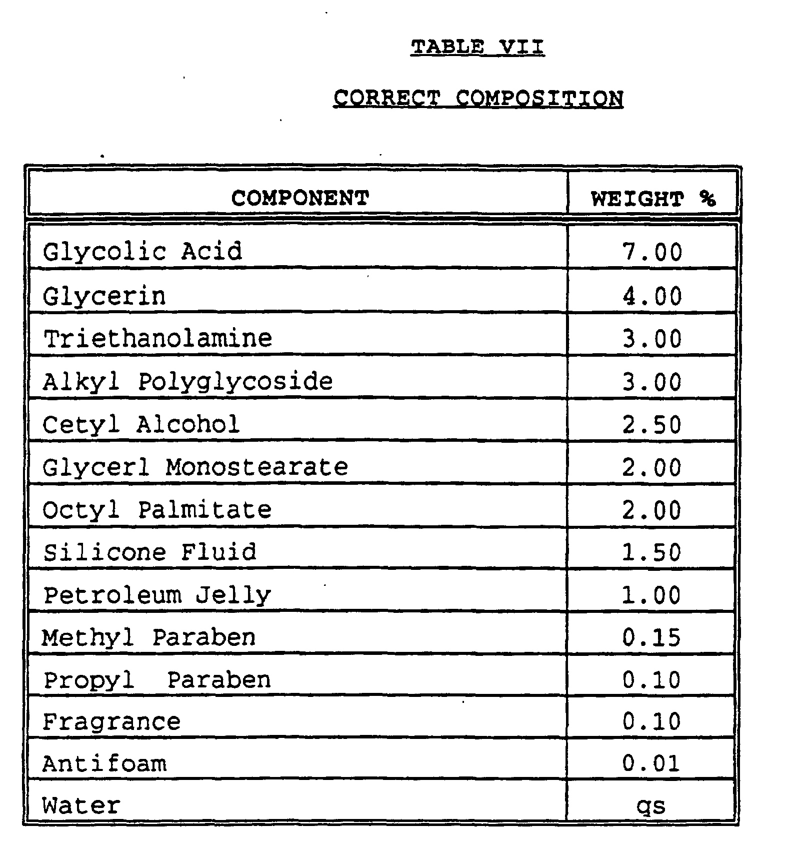

### EXAMPLE 5

Still a further pair of prevent and correct compositions according to the present invention are prepared from the following components.

**TABLE VIII**

| **PREVENT COMPOSITION** | |
|---|---|
| **COMPONENT** | **WEIGHT %** |
| Hexyl Laurate | 8.00 |
| Isopropyl Myristate | 3.00 |
| Stearic Acid | 3.00 |
| Propylene Glycol | 3.00 |
| Cyclomethicone | 3.00 |
| Octyl Methoxycinnamate | 2.00 |
| Panthenol | 1.00 |
| Butyl Methoxydibenzoylmethane | 1.00 |
| Disodium EDTA | 0.10 |
| Fragrance | 0.10 |
| Sodium Sorbate | 0.10 |
| Water | qs |

**TABLE IX**

| **CORRECT COMPOSITION** | |
|---|---|
| **COMPONENT** | **WEIGHT %** |
| Hexyl Laurate | 8.00 |
| Isopropyl Myristate | 3.00 |
| Stearic Acid | 3.00 |
| Propylene Glycol | 3.00 |
| Cyclomethicone | 3.00 |
| L-Lactic Acid | 2.00 |
| Panthenol | 1.00 |
| Ammonia (Aqueous 26 BE) | 0.30 |
| Disodium EDTA | 0.10 |
| Fragrance | 0.10 |
| Sodium Sorbate | 0.10 |
| Water | qs |

### EXAMPLE 6

Anhydrous formulations for a pair of prevent and correct compositions according to the present invention are prepared from the following components.

**TABLE X**

| **PREVENT COMPOSITION** | |
|---|---|
| **COMPONENT** | **WEIGHT %** |
| Cyclomethicone | 48.30 |
| Ethyl Alcohol | 24.70 |
| Isopropyl PPG-2 Isodeceth-7-carboxylate | 10.00 |
| Octyldimethyl PABA | 7.00 |
| Benzophenone-3 | 6.00 |
| Propylene Glycol Dicaprylate/Dicaprate | 4.00 |

**TABLE XI**

| **CORRECT COMPOSITION** | |
|---|---|
| **COMPONENT** | **WEIGHT %** |
| Cyclomethicone | 48.30 |
| Ethyl Alcohol | 24.70 |
| Isopropyl PPG-2 Isodeceth-7-carboxylate | 10.00 |
| Ammonium Glycolate | 7.00 |
| Propylene Glycol Dicaprylate/Dicaprate | 4.00 |
| Hydroxycaprylic Acid | 0.50 |

Although this invention has been described with reference to specific Examples and package embodiments, it will be apparent to one skilled in the art that various modifications may be made thereto which fall within the scope and purview of the invention.

## Claims

1. A cosmetic product to prevent and correct skin damage comprising:
a first composition comprising a sunscreen present in an effective amount to prevent UV radiation from penetrating the composition to reach a user's skin;
a second composition comprising a C₂-C₃₀ α-hydroxycarboxylic acid or salt thereof present in an effective amount to correct UV radiation induced skindamage;
a first container for storing the first composition; and
a second container for storing the second composition, the first and second containers being releasably joined together.

2. A product according to claim 1 wherein the first and second containers are each fitted with a means for coupling same to one another.

3. A product according to claim 2 wherein the means for coupling is a threaded screw.

4. A product according to claim 2 wherein the first and second containers are stacked one above the other and releasably locked together by the means for coupling.

5. A product according to claim 1 wherein outside walls of the first and second containers are colored differently from one another.

6. A product according to claim 5 wherein the color of the outside walls of the first container is predominantly white and the color of the outside walls of the second container is of a color predominantly other than white.

7. A product according to claim 1 wherein the sunscreen is an organic substance selected from the group consisting of para-amino benzoates; salicylates; cinnamates; benzophenones; coumarins; azoles; menthyl anthralinates; polyethylene; polyamides and mixtures thereof.

8. A product according to claim 1 wherein the sunscreen is present in an amount ranging from 0.1 to 25% by weight.

9. A product according to claim 1 wherein the C₂-C₃₀ α-hydroxycarboxylic acid is selected from the group consisting of lactic acid, glycolic acid, α-hydroxycaprylic acid and combinations thereof.

10. A product according to claim 1 wherein the C₂-C₃₀ α-hydroxycarboxylic acid or salt thereof is present in an amount from 0.01 to 15% by weight.

11. A product according to claim 1 wherein the first composition has at least ten separate components in common with those of the second composition.

12. A product according to claim 11 wherein the at least 10 separate components are present at about equal concentrations in the first and second compositions.

## Patentansprüche

1. Kosmetisches Produkt zur Prävention und Beseitigung von Hautschäden, umfassend:
eine erste Zusammensetzung, umfassend ein Sonnenschutzmittel, das in einer wirksamen Menge vorliegt, damit UV-Strahlung am Durchdringen der Zusammensetzung, um die Haut des Anwenders zu erreichen, gehindert wird; eine zweite Zusammensetzung, umfassend eine C₂-C₃₀-α-Hydroxycarbonsäure oder ein Salz davon, das in einer zur Beseitigung der durch UV-Strahlung induzierten Hautschädigung wirksamen Menge vorliegt; einen ersten Behälter zum Aufbewahren der ersten Zusammensetzung; und einen zweiten Behälter zum Aufbewahren der zweiten Zusammensetzung, wobei der erste und der zweite Behälter lösbar miteinander verbunden sind.

2. Produkt nach Anspruch 1, wobei der erste und der zweite Behälter jeweils mit einer Vorrichtung zum Verbinden miteinander ausgestattet sind.

3. Produkt nach Anspruch 2, wobei die Vorrichtung zum Verbinden ein Schraubgewinde ist.

4. Produkt nach Anspruch 2, wobei der erste und der zweite Behälter übereinander gestapelt sind und mit Hilfe der Verbindung lösbar miteinander verbunden sind.

5. Produkt nach Anspruch 1, wobei die Außenwände des ersten und des zweiten Behälters voneinander verschieden gefärbt sind.

6. Produkt nach Anspruch 5, wobei die Farbe der Außenwände des ersten Behälters vorwiegend Weiß ist und die Farbe der Außenwände des zweiten Behälters vorwiegend eine Farbe ist, die sich von Weiß unterscheidet.

7. Produkt nach Anspruch 1, wobei das Sonnenschutzmittel eine organische Substanz ist, ausgewählt aus der Gruppe, bestehend aus para-Aminobenzoaten; Salicylaten, Cinnamaten; Benzophenonen; Cumarinen; Azolen; Anthranilsäurementhylestern; Polyethylen; Polyamiden und Gemischen davon.

8. Produkt nach Anspruch 1, wobei das Sonnenschutzmittel in einer Menge im Bereich von 0,1 bis 25 Gewichtsprozent vorliegt.

9. Produkt nach Anspruch 1, wobei die C₂-C₃₀-α-Hydroxycarbonsäure aus der Gruppe, bestehend aus Milchsäure, Glycolsäure, α-Hydroxycaprylsäure und Kombinationen davon, ausgewählt ist.

10. Produkt nach Anspruch 1, wobei die C₂-C₃₀-α-Hydroxycarbonsäure oder ein Salz davon in einer Menge von 0,01 bis 15 Gewichtsprozent vorliegt.

11. Produkt nach Anspruch 1, wobei die erste Zusammensetzung mindestens zehn gesonderte Komponenten mit jenen der zweiten Zusammensetzung gemeinsam hat.

12. Produkt nach Anspruch 11, wobei die mindestens 10 gesonderten Komponenten bei etwa gleichen Konzentrationen in der ersten und der zweiten Zusammensetzung vorliegen.

## Revendications

1. Produit cosmétique pour prévenir et corriger la détérioration de la peau, comprenant :
une première composition comprenant un écran solaire présent dans une quantité efficace afin d'empêcher que les rayons UV ne pénètrent la composition pour atteindre la peau d'un utilisateur ;
une seconde composition comprenant un acide α-hydroxycarboxylique en C₂-C₃₀ ou un sel de celui-ci, présent dans une quantité efficace afin de corriger la détérioration de la peau due aux rayons UV;
un premier container pour stocker la première composition ; et
un second container pour stocker la seconde composition, le premier et le second container étant joints l'un à l'autre de telle sorte qu'ils puissent être séparés.

2. Produit selon la revendication 1, dans lequel le premier et le second container sont chacun fixés l'un à l'autre grâce à un moyen permettant de les coupler l'un à l'autre.

3. Produit selon la revendication 2, dans lequel le moyen de couplage est un pas de vis.

4. Produit selon la revendication 2, dans lequel le premier et le second container sont empilés l'un sur l'autre et fixés l'un à l'autre par le moyen de couplage de telle sorte qu'ils puissent être séparés.

5. Produit selon la revendication 1, dans lequel les parois externes du premier et du second container sont de couleurs différentes.

6. Produit selon la revendication 5, dans lequel la couleur des parois extérieures du premier container est blanche de façon et la couleur des parois extérieures du second container est de façon prédominante autre que le blanc.

7. Produit selon la revendication 1, dans lequel l'écran solaire est une substance organique sélectionnée à partir du groupe composé des para-amino benzoates ; des salicylates ;k des cinnamates; des benzophénones ; des coumarines ; des azoles ; des anthralinates menthyliques ; du polyéthylène ; des polyamides et des mélanges de ceux-ci.

8. Produit selon la revendication 1, dans lequel l'écran solaire est présent dans une quantité comprise dans la gamme allant de 0,1 à 25 % en masse.

9. Produit selon la revendication 1, dans lequel l'acide α-hydroxycarboxylique est sélectionné à partir du groupe composé de l'acide lactique, l'acide glycolique, l'acide α-hydroxycaprylique et des combinaisons de ceux-ci.

10. Produit selon la revendication 1, dans lequel l'acide α-hydroxycarboxylique en C₂ - C₃₀ ou le sel de celui-ci est présent dans une quantité allant de 0,01 à 15 % en masse.

11. Produit selon la revendication 1, dans lequel la première composition a au moins 10 composants qui sont les mêmes que eux de la seconde composition.

12. Produit selon la revendication 11, dans lequel les au moins dix composants séparés sont présents à des concentrations égales dans la première et dans la seconde composition.
